# FASCICULE DE BREVET EUROPEEN

(11) **EP 0 713 722 B1**
(45) Date de publication et mention de la délivrance du brevet: **27.10.1999**
(21) Numéro de dépôt: 95402501.1
(22) Date de dépôt: 08.11.1995
(51) Int. Cl.: B01F 3/02, A61M 16/00

(54) **Procédé et dispositif d'élaboration d'un mélange gazeux formé d'un gaz vecteur et d'un additif vaporisé**
Verfahren und Vorrichtung zur Herstellung eines Gasgemisches aus einem Trägergas und einem verdampften Zusatzmittel
Process and apparatus for preparing a gas mixture from a carrier gas and a vaporised additive

(30) Priorité: 23.11.1994 FR 9414037
(43) Date de publication de la demande: 29.05.1996
(73) Titulaire: TAEMA, F-92182 Antony Cédex (FR)
(72) Inventeur: Bloch, Nicholas, F-75014 Paris (FR); Ruton, Stéphane, F-78220 Viroflay (FR)
(74) Mandataire: Le Moenner, Gabriel

(56) Documents cités:
- EP-A- 0 231 513
- EP-A- 0 243 259
- EP-A- 0 280 846
- GB-A- 2 253 353
- US-A- 3 528 418

## Description

La présente invention est relative à un procédé et un dispositif pour l'élaboration d'un mélange gazeux en circulation et contenant une proportion définie d'un élément d'addition, notamment pour la production de mélanges gazeux anesthésiques.

Le document EP-A-0.231.513 décrit un dispositif d'obtention de mélanges gazeux, notamment anesthésiques, formés d'un gaz vecteur dans lequel on a injecté une quantité dosée d'un additif. Le dispositif comprend une enceinte fermée, maintenue à une pression et une température constantes, cette pression et cette température étant telles que l'additif y est sous forme gazeuse. Un passage de sortie relie l'enceinte à un conduit dans lequel est envoyé le gaz vecteur. Ce passage est équipé de moyens de dosage constitués d'une vanne qui est ouverte cycliquement et/ou d'une vanne constituant un étranglement réglable. L'enceinte est reliée à un récipient fermé contenant de l'additif à l'état liquide, de telle façon que, chaque fois que de l'additif sort de l'enceinte à travers le passage de sortie, une masse égale de l'additif liquide pénètre dans l'enceinte pour y être vaporisée. Pour cela, il suffit que le niveau du liquide dans le récipient soit plus bas que l'enceinte, que la liaison entre le récipient et l'enceinte soit assurée par un tube qui plonge dans le liquide, et que la pression au-dessus du liquide soit définie en fonction de la pression qu'on désire maintenir dans l'enceinte.

Dans le dispositif décrit dans le document cité, le réglage du débit d'additif à travers le passage de sortie, c'est-à-dire finalement la teneur en additif du mélange, est opéré en agissant sur la fréquence d'ouverture de la vanne dans le passage de sortie.

Ce mode opératoire a pour inconvénient qu'il ne présente pas une sécurité intrinsèque : si la vanne de réglage vient à se bloquer en position ouverte, ou fermée, au cours d'une anesthésie, les conséquences peuvent être immédiatement dramatiques.

On observera aussi que la pression dans l'enceinte doit toujours être largement inférieure à celle qui correspond à l'ébullition du liquide à la température dans cette enceinte. En effet, si la pression dans l'enceinte montait au-delà de cette valeur limite, les moyens de dosage commenceraient à débiter l'additif sous forme liquide et non plus sous forme gazeuse. Il en résulterait une augmentation très brutale, et éventuellement très dangereuse, de la teneur en additif du mélange. Un tel accident pourrait se produire à la suite d'un arrêt des moyens de chauffage de l'enceinte. Comme on opère à pression constante, il paraît donc nécessaire que les moyens de sécurité bloquent les moyens de dosage avant que l'additif liquide ait pu les atteindre. Dans ce cas, un arrêt du chauffage entraînera une chute brusque de la teneur en additif du mélange.

L'appareillage doit donc être associé à des dispositifs d'alarme et de sécurité à réponse très rapide, qui augmentent sa complication et son prix de revient.

Un autre inconvénient du dispositif décrit dans le document susmentionné résulte du fait que le réglage du débit d'additif est fait en agissant sur la fréquence d'ouverture de la vanne de dosage. Pour les faibles teneurs, on cite une fréquence pouvant descendre jusque 0,1 Hz, soit une impulsion toutes les dix secondes. Des fréquences aussi basses peuvent imposer la présence, en aval, d'un système d'homogénéisation, ce qui est une complication supplémentaire.

La présente invention a pour but de remédier à ces inconvénients, et de fournir un procédé d'obtention de mélange gazeux en circulation sûr, simple et efficace, dans lequel un défaut de fonctionnement des moyens de régulation ait une incidence réduite sur la teneur du mélange, et qui fournisse directement un mélange à teneur constante.

A cette fin, selon l'invention, le procédé d'élaboration d'un mélange gazeux formé d'un gaz vecteur et d'au moins un additif vaporisé, comprenant les étapes de vaporiser des quantités dosées de l'additif contenu sous forme liquide dans un récipient d'où il est expulsé vers un vaporiseur par du gaz de chasse sous pression introduit dans le récipient, est caractérisé en ce qu'on règle le débit d'additif introduit dans le gaz vecteur en agissant sur la pression du gaz de chasse.

Des moyens pratiques, pour réguler la pression de gaz sont un détendeur dans le cas où le gaz provient d'un récipient pressurisé, ou un régulateur dans le cas d'une pompe. Dans l'un et l'autre cas, il s'agit de matériels de grande fiabilité. En outre, si l'alimentation en gaz est interrompue accidentellement, les gaz contenus dans le récipient et dans l'enceinte de chauffage constituent un volume tampon. En conséquence, la pression dans le vaporiseur ne varie que lentement en cas d'incident, de même que le débit d'additif. Cela laisse au personnel le temps d'intervenir pour prendre les mesures nécessaires. Pour une plus grande sécurité, un réservoir tampon complémentaire peut être relié à la partie supérieure du récipient.

D'un autre côté, puisque la pression est le paramètre de réglage, il est aisé de coupler les moyens de commande de la pression avec un capteur sensible à la température de l'enceinte de vaporisation, de façon que, en cas de baisse indésirée de la température de l'enceinte, on abaisse la pression, soit en fonction de ladite température, soit selon une loi fixée à l'avance, de façon à maintenir toujours la pression dans l'enceinte à une valeur inférieure à la pression où le liquide se vaporise à la température instantanée de l'enceinte.

Enfin, le fait que le débit d'additif est contrôlé en fonction d'une pression permet de s'affranchir d'une vanne à fréquence variable entre le vaporiseur et la conduite de distribution mélange. On peut ainsi utiliser une simple vanne, ou un étranglement, qui, pour une pression fixe donnée, laisse passer un débit sensiblement constant dans le temps, ce qui permet d'obtenir directement un mélange à teneur constante en additif.

Suivant une variante préférée, le moyen de contrôle de débit, de l'additif vaporisé, comporte un jeu d'étranglements de différentes sections, et on met en action ou hors d'action un ou plusieurs de ces étranglements en fonction des conditions générales d'exécution.

Avantageusement, on commute un ou plusieurs étranglements dudit jeu pour un ou plusieurs autres étranglements lorsque la pression commandée s'approche à une distance déterminée d'une pression limite choisie à l'avance.

Pour des raisons de sécurité, il est préférable de prévoir des contrôles de la composition de mélange gazeux, et on déclenche une alarme lorsque la teneur en additif franchit des limites fixées à l'avance. Les contrôles peuvent être continus ou périodiques, avec une fréquence fixée à l'avance.

Suivant un mode opératoire optionnel, pour obtenir une plus grande régularité de la composition du mélange, les moyens de contrôle de la composition du mélange gazeux font avantageusement partie d'un système du type "à boucle fermée", c'est-à-dire qu'on modifie éventuellement la pression du gaz envoyé au récipient en fonction du résultat des contrôles.

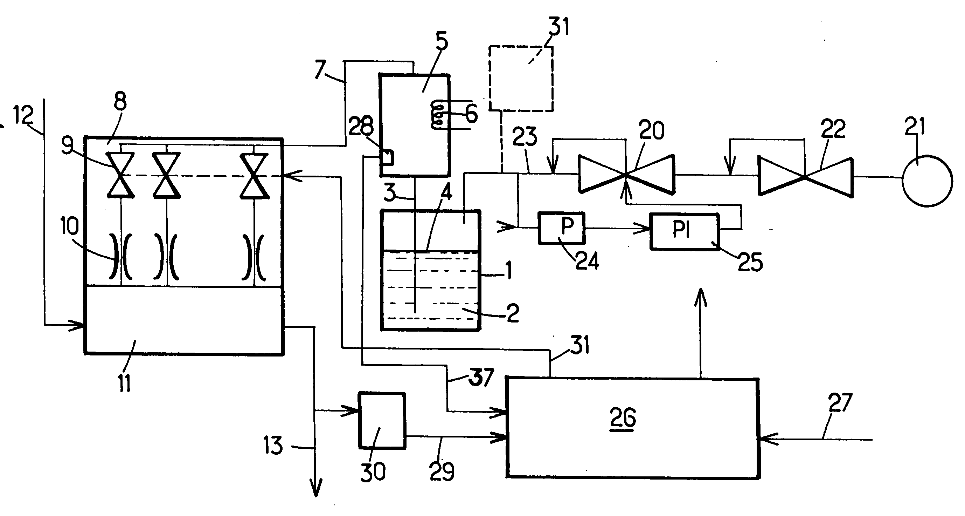

L'invention va maintenant être exposée de façon plus détaillée à l'aide d'un exemple pratique, illustré par la figure unique, qui est un schéma d'un appareillage selon l'invention.

Le dispositif utilisé à titre d'exemple comprend : Un récipient préssurisable 1 contenant une charge d'additif liquide 2, un tube plongeur 3, qui traverse la paroi du récipient 1, plonge au-dessous du niveau 4 de l'additif liquide et conduit à une enceinte de chauffage 5 d'un vaporiseur, maintenue à une température convenable par une résistance chauffante 6, et un conduit de sortie 7, reliant l'enceinte de chauffage à un ensemble de contrôle de débit 8.

Cet ensemble 8 comprend plusieurs passages situés en parallèle, comportant chacun, en série, une vanne 9 et un étranglement fixe 10. En jouant sur le nombre de vannes 9 ouvertes ou fermées, et/ou la section de passage des étranglements 10, on peut obtenir une large gamme de débits. Les étranglements 10 débouchent dans un collecteur-mélangeur 11, dans lequel du gaz porteur fourni par un conduit d'amenée 12 pénètre par une extrémité, alors que le mélange sort par une extrémité opposée pour être distribué par un conduit de distribution 13.

Les moyens pour commander la pression à l'intérieur du récipient 1 comprennent essentiellement une vanne pilotée 20 disposée, en aval d'un détendeur 22, dans une ligne 23 reliant le récipient 1 à une source de gaz sous pression 21. La source de gaz sous pression 21 peut être quelconque, à condition que la pression soit dans des limites convenables, ici 2 à 8 x 10⁵ Pa. Dans la pratique, on choisit, de préférence, le réseau d'oxygène d'un ensemble hospitalier, qui présente l'avantage d'être accessible en de très nombreux points.

Sur l'extrémité du conduit 23 qui débouche à la partie supérieure du récipient 1, est branché un capteur de pression 24, qui indique par conséquent la pression régnant à l'intérieur du récipient 1. Le capteur 24 est relié à un régulateur de pression 25, qui est par exemple un régulateur du type "Sentronic" vendu par la société française Joucomatic, mais qui peut être un régulateur de n'importe quel type ayant des performances comparables.

Un calculateur électronique 26, à microprocesseur et mémoires ROM et RAM, établit la valeur de la pression commandée qui est transmise au régulateur 25. Le calculateur 26 reçoit par une entrée 27 une valeur de consigne de la composition du mélange à produire. Une ligne d'entrée 37 relie une entrée du calculateur à un capteur de température 28 installé dans l'enceinte de chauffage 28 et une autre ligne d'entrée 29 transmet au calculateur 26 des signaux de composition du mélange sortant, émis par un analyseur 30 relié au conduit de distribution 13.

On a représenté en tirets un réservoir tampon 31 qui peut être prévu pour ralentir la chute de pression dans le récipient 1 en cas d'arrêt brusque intempestif de la source de pression 21.

Une première série d'essais a été faite avec l'appareillage qu'on vient de décrire. Dans ces essais, le gaz porteur est l'air et l'additif de l'halothane. La température dans l'enceinte de chauffage 5 est de 80°C. On a obtenu les résultats suivants :

| Débit gaz vecteur (l/m) | Concentration (% vol) | P liquide (kPa) | Débit vapeur (ml/mn) |
|---|---|---|---|
| 0,5 | 3 | 11,1 | 15 |
| 10 | 0,2 | 17,4 | 20 |
| 15 | 0,4 | 80,0 | 60 |
| 20 | 1 | 74,4 | 200 |

On constate que le procédé et l'appareillage selon l'invention permettent de couvrir une très large gammes de débits et de concentration.

## Revendications

1. Procédé d'élaboration d'un mélange gazeux formé d'un gaz vecteur et d'au moins un additif vaporisé, comprenant les étapes de vaporiser des quantités dosées de l'additif contenu sous forme liquide dans un récipient d'où il est expulsé vers un vaporiseur par du gaz de chasse sous pression introduit dans le récipient, caractérisé en ce qu'on règle le débit d'additif introduit dans le gaz vecteur en agissant sur la pression du gaz de chasse.

2. Procédé selon la revendication 1, caractérisé en ce qu'on contrôle la composition du mélange gazeux, et on déclenche une alarme lorsque la teneur en additif franchit des limites fixées à l'avance.

3. Procédé selon la revendication 1 ou 2, caractérisé en ce qu'on procède à des contrôles de la composition du mélange gazeux et on règle la pression du gaz de chasse envoyé dans le récipient en fonction du résultat de ces contrôles.

4. Procédé selon l'une des revendications 1 à 3, caractérisé en ce qu'on règle la pression du gaz de chasse en fonction d'au moins un paramètre mesuré du circuit de délivrance d'additif.

5. Procédé selon la revendication 4, caractérisé en ce qu'un paramètre est la pression dans le récipient.

6. Procédé selon la revendication 4 ou 5, caractérisé en ce qu'un paramètre est une température dans le vaporiseur.

7. Procédé selon l'une des revendications 4 à 6, caractérisé en ce qu'un paramètre est la proportion d'un composant du mélange gazeux.

8. Procédé selon l'une des revendications 1 à 7, caractérisé en ce que le gaz de chasse est l'oxygène.

9. Dispositif d'élaboration d'un mélange gazeux formé d'un gaz vecteur et d'au moins un additif vaporisé, comprenant :
- un récipient (1) contenant l'additif sous forme liquide ;
- des moyens (20-26) pour envoyer un gaz de chasse sous pression dans le récipient (1);
- un circuit de distribution d'additif vers un circuit de gaz vecteur (12, 13), circuit comprenant, en série, un tube plongeur (3) s'étendant dans le récipient, un vaporiseur (5), et un moyen de contrôle de débit (8) de l'additif vaporisé,
caractérisé en ce que les moyens pour envoyer le gaz de chasse Sous pression dans le récipient (1) comprennent un moyen de vanne pilotée (20) disposé dans une conduite (23) entre une source (21,22) de gaz de chasse sous pression et le récipient (1) et actionné en fonction d'un signal représentatif de la pression dans le récipient.

10. Dispositif selon la revendication 9, caractérisé en ce qu'il comprend au moins un détecteur d'un paramètre dans le circuit d'additif vaporisé (7, 13), couplé à un calculateur (26) fournissant des signaux de commande au moyen de vanne pilotée en fonction du paramètre détecté.

## Claims

1. Process for preparing a gas mixture formed from a carrier gas and from at least one vaporized additive, comprising the steps of vaporizing metered amounts of the additive contained in liquid form in a container from which it is expelled towards a vaporizer by expulsion gas under pressure introduced into the container, characterized in that the additive flow rate introduced into the carrier gas is adjusted by varying the pressure of the expulsion gas.

2. Process according to Claim 1, characterized in that the composition of the gas mixture is checked and an alarm is triggered when the additive content departs from preset limits.

3. Process according to Claim 1 or 2, characterized in that checks of the composition of the gas mixture are made and the pressure of the expulsion gas sent into the container is adjusted depending on the result of these checks.

4. Process according to one of Claims 1 to 3, characterized in that the pressure of the expulsion gas is adjusted depending on at least one measured parameter of the additive delivery circuit.

5. Process according to Claim 4, characterized in that one parameter is the pressure in the container.

6. Process according to Claim 4 or 5, characterized in that one parameter is a temperature in the vaporizer.

7. Process according to one of Claims 4 to 6, characterized in that one parameter is the proportion of one component of the gas mixture.

8. Process according to one of Claims 1 to 7, characterized in that the expulsion gas is oxygen.

9. Apparatus for preparing a gas mixture formed from a carrier gas and from at least one vaporized additive, which comprises:
- a container (1) which contains the additive in liquid form;
- means (20-26) for sending an expulsion gas under pressure into the container (1);
- a circuit for delivering the additive into a carrier-gas circuit (12, 13), which delivery circuit comprises, in series, a dip tube (3) extending into the container, a vaporizer (5) and a means (8) for controlling the flow rate of the vaporized additive,
characterized in that the means for sending the expulsion gas under pressure into the container (1) comprise a controlled-valve means (20) which is placed in a line (23) between a supply (21, 22) of expulsion gas under pressure and the container (1) and is actuated depending on a signal representative of the pressure in the container.

10. Apparatus according to Claim 9, characterized in that it comprises at least one detector of a parameter in the vaporized-additive circuit (7, 13), coupled to a computer (26) which supplies control signals to the controlled-valve means depending on the parameter detected.

## Patentansprüche

1. Verfahren zur Herstellung eines Gasgemischs aus einem Trägergas und mindestens einem verdampften Zusatzstoff, bei dem man abgemessene Mengen des in einem Behältnis in flüssiger Form enthaltenen Zusatzstoffs, der von dort durch unter Druck stehendes, in das Behältnis eingespeistes Treibgas in Richtung eines Vergasers ausgestoßen wird, verdampft, dadurch gekennzeichnet, daß man den in das Trägergas eingetragenen Zusatzstoffdurchsatz durch Einstellung des Treibgasdrucks reguliert.

2. Verfahren nach Anspruch 1, dadurch gekennzeichnet, daß man die Zusammensetzung des Gasgemischs kontrolliert und einen Alarm auslöst, wenn der Zusatzstoffgehalt von vorbestimmten Grenzwerten abweicht.

3. Verfahren nach Anspruch 1 oder 2, dadurch gekennzeichnet, daß man Kontrollen der Zusammensetzung des Gasgemischs vornimmt und den Druck des in das Behältnis geleiteten Treibgases in Abhängigkeit vom Ergebnis dieser Kontrollen reguliert.

4. Verfahren nach einem der Ansprüche 1 bis 3, dadurch gekennzeichnet, daß man den Treibgasdruck in Abhängigkeit von mindestens einem Meßparameter des Zusatzstoffzufuhrkreislaufs reguliert.

5. Verfahren nach Anspruch 4, dadurch gekennzeichnet, daß man als einen Parameter den Druck im Behältnis nimmt.

6. Verfahren nach Anspruch 4 oder 5, dadurch gekennzeichnet, daß man als einen Parameter eine Temperatur im Vergaser nimmt.

7. Verfahren nach einem der Ansprüche 4 bis 6, dadurch gekennzeichnet, daß man als einen Parameter den Anteil eines Bestandteils des Gasgemischs nimmt.

8. Verfahren nach einem der Ansprüche 1 bis 7, dadurch gekennzeichnet, daß man als Treibgas Sauerstoff verwendet.

9. Vorrichtung zur Herstellung eines Gasgemischs aus einem Trägergas und mindestens einem verdampften Zusatzstoff mit:
- einem Behältnis (1), das den Zusatzstoff in flüssiger Form enthält;
- Mitteln (20-26) zum Einleiten eines unter Druck stehenden Treibgases in das Behältnis (1);
- einem Kreislauf zur Verteilung des Zusatzstoffs zu einem Trägergaskreislauf (12, 13), enthaltend der Reihe nach ein sich in das Behältnis erstreckendes Tauchrohr (3), einen Vergaser (5) und ein Mittel zur Kontrolle des Durchsatzes (8) des verdampften Zusatzstoffs,
dadurch gekennzeichnet, daß die Mittel zum Einleiten des unter Druck stehenden Treibgases in das Behältnis (1) ein in einer Leitung (23) zwischen einer Quelle (21, 22) von unter Druck stehendem Treibgas und dem Behältnis (1) angeordnetes und in Abhängigkeit von einem Behältnisdrucksignal betätigtes Regelventilmittel (20) umfassen.

10. Vorrichtung nach Anspruch 9, dadurch gekennzeichnet, daß sie mindestens einen Detektor für einen Parameter im Kreislauf des verdampften Zusatzstoffs (7, 13) umfaßt, der an einen Rechner (26) angeschlossen ist, welcher Steuersignale mit Hilfe eines Regelventils in Abhängigkeit vom detektierten Parameter liefert.
